Europäisches Patentamt

European Patent Office

Office européen des brevets

(11) Publication number: **0 276 058**
**A1**

# (12) EUROPEAN PATENT APPLICATION

(21) Application number: **88300123.2**

(22) Date of filing: **08.01.88**

(51) Int. Cl.⁴: **A 61 K 49/00**
**G 01 N 24/08**

(30) Priority: **09.01.87 GB 8700431**

(43) Date of publication of application:
**27.07.88 Bulletin 88/30**

(84) Designated Contracting States:
**AT BE CH DE FR GB IT LI LU NL SE**

(71) Applicant: **AMERSHAM INTERNATIONAL plc**
**Amersham Place**
**Little Chalfont Buckinghamshire HP7 9NA (GB)**

(72) Inventor: **Sadler, Peter John**
**40 Albion Road Pitstone**
**Leighton Buzzard Bedfordshire LU7 9AY (GB)**

**McEwen, Andrew Bruce**
**4 Nightingale Court 54 Thrale Road**
**Streatham SW16 1NY (GB)**

(74) Representative: **Pennant, Pyers et al**
**Stevens, Hewlett & Perkins 5 Quality Court Chancery**
**Lane**
**London, WC2A 1HZ (GB)**

(54) Contrast agents for NMR spectroscopy.

(57) A method of detecting the interaction between a metabolite and an NMR contrast agent comprising paramagnetic metal ions, present together in a biological fluid. The method comprises subjecting a sample of the fluid to NMR specrtoscopy and comparing the results to those obtained for a sample of the biological fluid from which the contrast agent is substantially absent.

The NMR contrast agents, when added to the biological material under investigation, cause specific changes in the chemical shift or relaxation times of specific resonances in the spectra. They can thus be of assistance in the assignment of particular peaks and in the general interpretation of the spectra.

The method can be used with a variety of biological or body fluid, such as urine and plasma serum, and can either be performed in vivo or on extracted samples of these fluids.

EP 0 276 058 A1

## Description

## Contrast Agents for NMR Spectorscopy

This invention relates to contrast agents comprising paramagnetic metal ions and their use in nuclear magnetic resonance (NMR) spectorscopy. The contrast agents interact with metabolites present in biological fluids and these interactions can be detected in NMR spectra.

Modern NMR apparatus can detect signals from a variety of nuclei, in particular $^1H$, $^{13}C$, $^{19}F$ and $^{31}P$, both from isolated samples of tissues and fluids and also from intact living organisms. $^1H$ NMR signals from water can be mapped spatially to form an NMR image. With in vivo spectroscopy, the achievable resolution between resonances for nuclei of the same type in different magnetic environments is low because of the low magnetic fields used and poor field homogeneity over the sample volume. The detection of $^1H$ NMR signals from metabolites in the presence of a water signal which is more than 10,00 times as intense presents a major difficulty. Improvements in the design of instrumentation have however occurred recently and, with the use of higher fields and solvent suppression techniques, resolved resonances can now be detected from localised areas of intact living animals. Well resolved high resolution spectra can also be obtained from a variety of extracted body fluids, including urine, plasma and cerebrospinal fluid. Nicholson et al., Biochem. J. (1984) 217, 365-375 and Bales et al., Clin. Chem. (1984) 30, 426-432 described spectra of this type. Such spectra clearly represent potential sources of important biochemical information of medical relevance.

It is noted in Clin. Chem. (1984) 30, 426-432 that the presence of paramagnetic metal ions causes a broadening of the resonances (peaks) of certain metabolites in NMR spectra and, in extreme cases, can make them unobservable. The presence of such ions has heretofore been considered a problem since they can lead to difficulties in correctly assigning peaks in the spectra to specific metabolites.

Difficulties also frequently arise when the spectra are crowded, perhaps containing many overlapping peaks, and so poorly resolved that spin-spin couplings are obscured. This can make it impossible to assign peaks in the spectra to specific nuclei of metabolites and the information which can be gleaned from the spectra is significantly reduced.

This invention is concerned with the use of contrast agents which, when added to the biological material under investigation, cause specific changes in the chemical shift or relaxation times of specific resonances so as to assist in their assignment.

According to the present invention there is provided a method of detecting the interaction between a metabolite and an NMR contrast agent comprising paramagnetic metal ions, present together in a biological fluid, which method comprises subjecting a sample of said biological fluid to NMR spectroscopy and comparing the results to those obtained for a sample of the biological fluid from which the contrast agent is substantially absent.

The contrast agent can be used in any of the aforementioned types of NMR spectroscopy, and is considered to be most useful in high resolution $^1H$ NMR spectroscopy. The method of the invention is applicable for use with biological fluids both in vitro and in vivo, and can be used to provide contrast in both one dimensional and two dimensional NMR spectra.

Paramagnetic metal ions whose presence can cause shifts of NMR resonances include $Eu^{3+}$ and $Pr^{3+}$, while those which have the effect of shortening relaxation times (broadening agents) include $Mn^{2+}$ and $Gd^{3+}$. Other metal ions can cause both shifts and broadenings, such as $Fe^{3+}$, $Co^{2+}$, $Ni^{2+}$ and $Dy^{3+}$. The use of simple salts of these metal ions is not always possible because of their toxicity and the fact that free metal ions can precipitate out in certain tissue extracts. In such cases, the paramagnetic metal ions may be used in the form of chelated complexes.

Complexes of the paramagnetic metals may be formed using any suitable chelating agent, the use of EDTA or DTPA being preferred. The chelate reduces toxicity and solubility problems and the interaction with the metabolites which are of interest will be through the formation of ternary complexes. The ternary complex association is strongly influenced by the choice of both metal and complexing ligand.

Specific paramagnetic metal ions and complexes thereof have to be selected for investigations with regard to the target metabolite. Those metabolites of interest include citrate, adenosine triphosphate (ATP), alanine and glycine, but obviously there are many others. The interaction between the metabolite and the paramagnetic metal affects the spectrum produced when the sample is subjected to NMR spectroscopy. The resonances normally attributable to the metabolite may be shifted or broadened, or may even completely disappear. It will be appreciated that this effect can be of great benefit in the interpretation of NMR spectra. It can be used to conclusively identify the presence of particular metabolites and, in the case of very crowded spectra, the removal of some of the peaks will enable the remaining resonances to be studied more carefully so that they can be correctly assigned.

In use, the paramagnetic ions are added (in vitro) or injected (in vivo) at levels ranging from very small ($\mu m$) doses to millimolar doses as required. Two samples would be taken and subjected to NMR spectorscopy, one in the absence of the contrast agent and the other in its presence. The resulting spectra are then compared to see the effects of the interaction of the paramagnetic metal with the target metabolite. The method can be used with a variety of biological or body fluids, including urine, plasma serum, amniotic fluid, cerebrospinal fluid and the aqueous humour from the eye. The method can either be performed in vivo or on extracted samples of these fluids.

In addition to the aforementioned uses, namely the unambiguous identification of those components whose lines disappear in NMR spectra when the paramagnetic metals are added and the removal of unwanted components in order to improve the discrimination of other components in the spectrum, other uses are also envisaged. For example, the method may be used in an analytical role to detect the presence of paramagnetic metals or perhaps to enable the passage of such metals through the body to be followed. Comparing the effects of the presence of the paramagnetic metal ions on spectra from sick patients with those from normal healthy persons may have a direct diagnostic role.

The invention will now be illustrated by the following examples, which clearly demonstrate the potential use of paramagnetic metals to simplify NMR spectra for various biological fluids in vitro and the possible extension to use in vivo.

Example 1

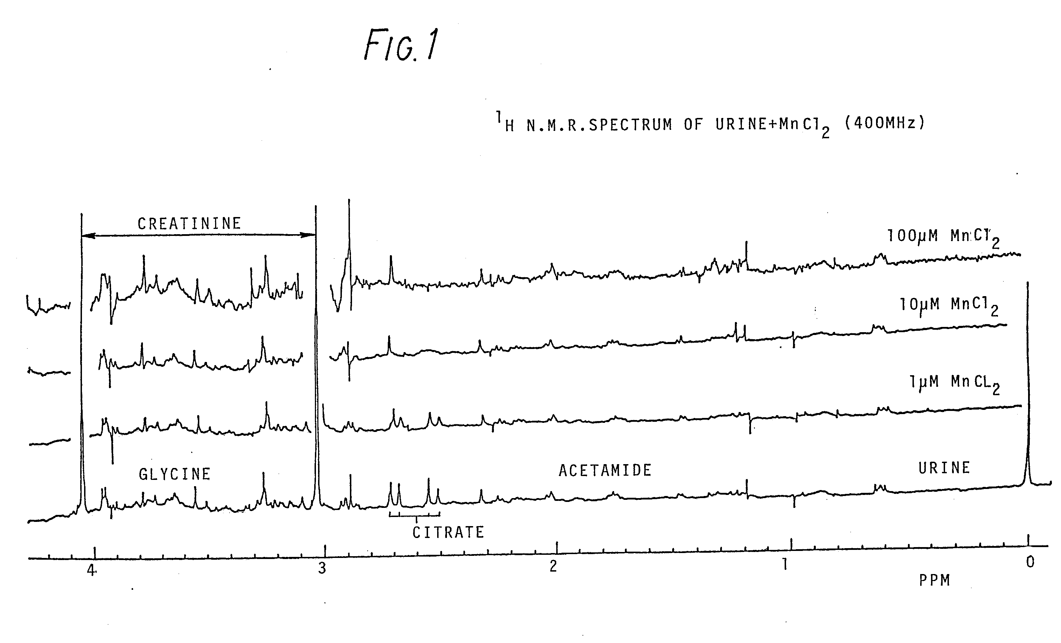

0.45 ml of a urine sample from a normal male adult after an overnight fast was placed in a 5mm diameter NMR tube and 0.05ml $D_2O$ was added as lock signal. The high resolution $^1H$ NMR spectrum was recorded at 400 MHz using continuous irradiation to suppress the $H_2O$ peak. On addition of $MnCl_2$ (at a level of 1, 10 and 100 $\mu m$), a specific broadening of the $^1H$ resonance for citrate at 2.639 ppm was observed. The results are shown in Figure 1. By using a Hahn spin echo pulse sequence $90°-\gamma-180°$ ($\gamma$-collect free induction decay), usually accumulated about 48 times, it was possible to effect the complete disappearance of citrate signals from the spectrum. The only other resonances affected, to a lesser degree, were those of glycine and that of an unidentified metabolite at 2.88 ppm.

This shows that the addition of a small amount of a simple salt of manganese ($MnCl_2$) to a sample of urine causes specific broadening, and even the complete disappearance, of resonances in NMR spectra.

Example 2

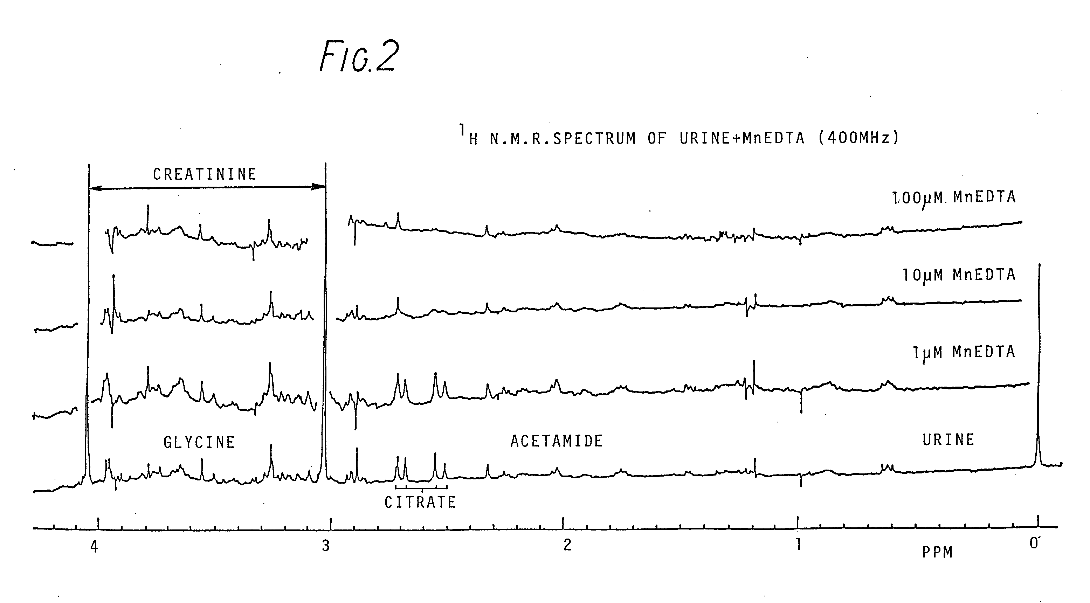

The method of Example 1 was repeated this time using 1,10 and 100 $\mu m$ of MnEDTA: N-methyl glucamine salt in place of $MnCl_2$. The results are shown in Figure 2. Once again a specific broadening of the $^1H$ resonance for citrate was observed.

This shows that the addition of a small amount of a chelated form of manganese (MnEDTA) has a similar effect to the simple salt of Example 1. The use of the MnEDTA complex would obviously be preferred in vivo.

Example 3

(a) Synthesis of Nd(III)EDTA.

8.08g of neodymium oxide and 14.03g of EDTA were suspended in 250ml $H_2O$. The mixture was heated, with stirring, at 95°C for 48 hours. The suspension turned pale violet during the course of the reaction. The produce was filtered off and washed with cold water, ethanol and ether, before dried at 100°C overnight. Yield 16.52g, 76.49%.
Calculated for C10 H15 N2 O9 Nd    C 26.60, H 3.36, N 6.20
Found    C 26.32, H 3.57, N 6.21
Complex analyses as Nd(III)EDTA.$H_2O$(EDTA monoprotonated)

(b) Effect of NdEDTA on $^{31}P$ resonances of ATP.

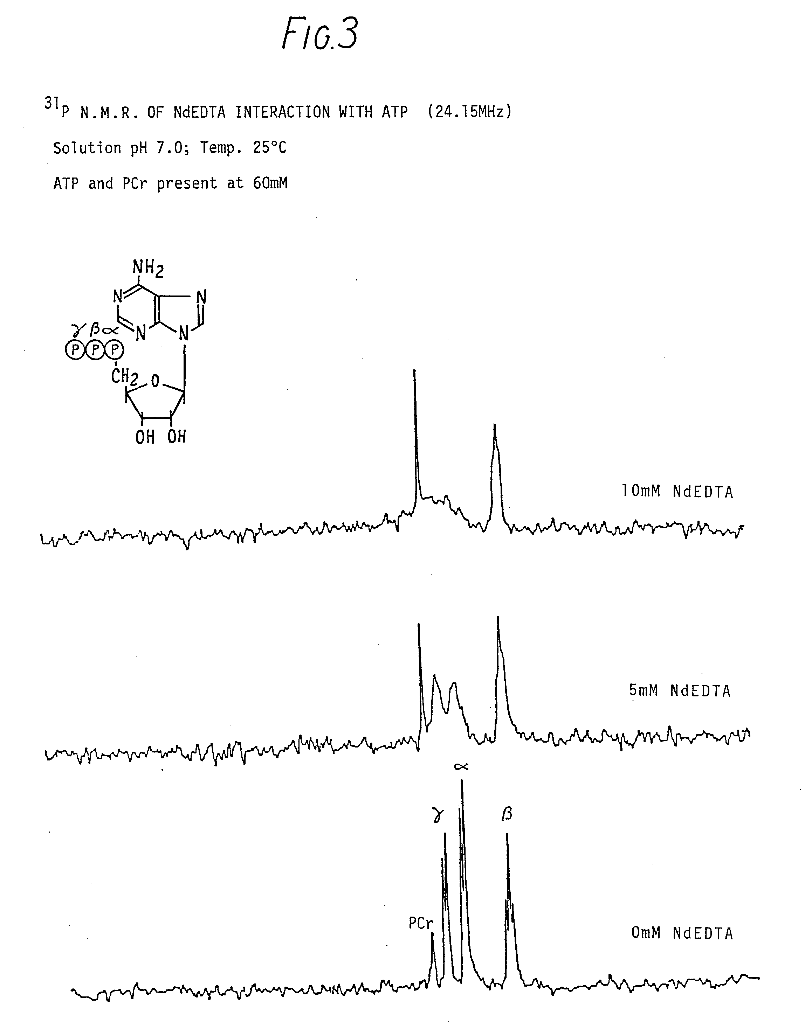

NdEDTA was dissolved at pH 7.0 and a concentration of 0.1M by the addition of N-methyl glucamine. A standard solution of ATP and PCr (phosphocreatine), each 60mM, was prepared by dissolving both in water, the pH was adjusted to 7.0 by the addition of NaOH. The $^{31}P$ NMR spectrum was obtained at 24.15 MHZ in 256 scans. NdEDTA was then added from a pipette to give a final concentration of 5mM metal complex. On rerunning the spectra it was observed that the $\gamma$ and $\alpha$ resonances were severely broadened whilst the resonance for the $\beta$ phosphorus was relatively unaffected. On the other hand the resonance for phosphocreatine remained sharp. Addition of NdEDTA at 10mM resulted in complete disappearance of the $\gamma$ and $\alpha$ resonances from the spectrum leaving the $\beta$ resonance and PCr resonance relatively unaffected. The results are shown in Figure 3. The broadening effects were unexpected for $Nd^{3+}$. In previously reported work, the main effects on $^{31}P$ resonances of ATP after addition of $Nd(NO_3)_3$ were shifts of resonances.

This demonstrates that the addition of a complex of neodymium (NdEDTA) will simplify $^{31}P$ NMR spectra. Such an effect would be particularly useful for in vivo work.

Example 4

Effect of NdEDTA on $^1H$ resonances.

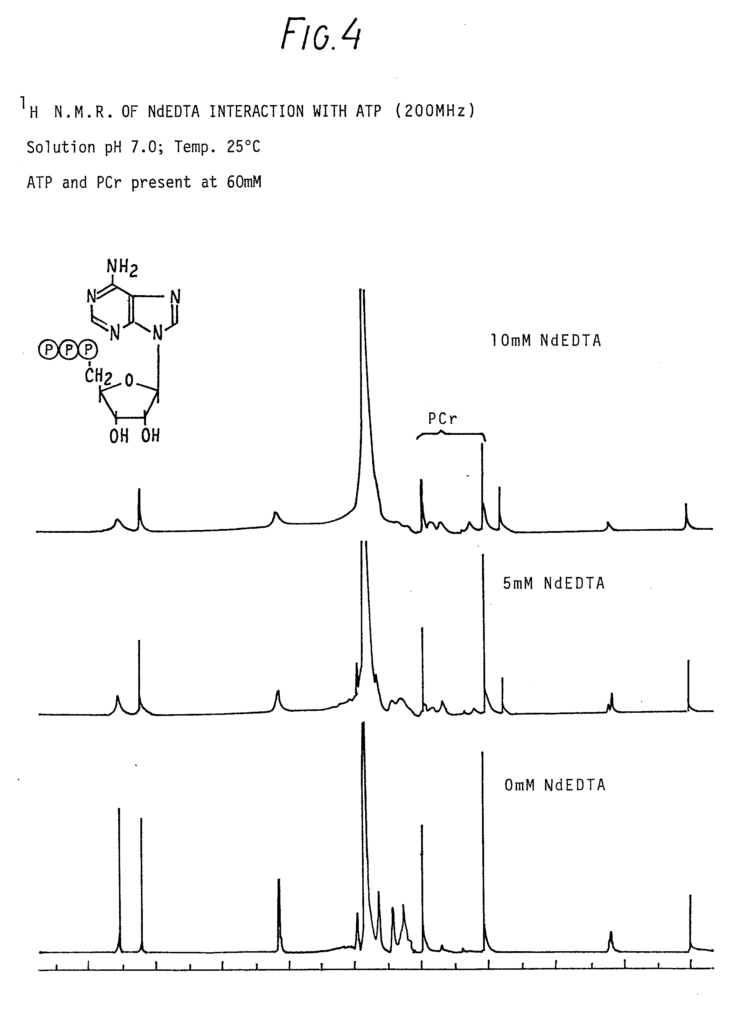

Stock solutions NdEDTA and ATP/PCr were prepared as in Example 3. The $^1H$ NMR spectrum was recorded at 200 MHZ without NdEDTA being present. NdEDTA was then added at a final concentration of 5mM and 10mM as in the previous example. The resonances for the sugar ring protons broadened and disappeared as the level of NdEDTA was increased and the aromatic proton at C-2 was severely broadened whereas that at C-8 remained sharp. The resonances for PCr were unaffected by the addition of NdEDTA. The results are shown in Figure 4.

This shows that the addition of a complex of neodymium (NdEDTA) can cause selective spectral contrast in $^1H$ NMR spectra.

**Claims**

1. A method of detecting the interaction between a metabolite and an NMR contrast agent comprising paramagnetic metal ions, present together in a biological fluid, which method comprises subjecting a sample of said biological fluid to NMR spectroscopy and comparing the results to those obtained for a sample of the biological fluid from which the contrast agent is substantially absent.

2. A method as claimed in claim 1, wherein the paramagnetic metal ions are manganese or neodymium.

3. A method as claimed in claim 1 or claim 2, wherein the paramagnetic metal ions are chelated in the form of a complex.

4. A method as claimed in claim 3, wherein the chelating agent used to prepare the complex is EDTA,

5. A method as claimed in any one of the preceding claims, wherein the biological fluid is urine.

6. A method as claimed in any one of the preceding claims, wherein the metabolite is citrate.

7. A method as claimed in any one of the preceding claims, wherein the NMR spectorscopy used is high resolution $^1$H NMR.

8. A method as claimed in any one of the preceding claims and which is performed in vitro.

9. A method as claimed in any one of claims 1 to 7 and which is performed in vivo.

FIG.1

$^1$H N.M.R.SPECTRUM OF URINE+MnCl$_2$ (400MHz)

*FIG.2*

$^1$H N.M.R.SPECTRUM OF URINE+MnEDTA (400MHz)

0276058

# FIG.3

$^{31}$P N.M.R. OF NdEDTA INTERACTION WITH ATP  (24.15MHz)

Solution pH 7.0; Temp. 25°C

ATP and PCr present at 60mM

10mM NdEDTA

5mM NdEDTA

OmM NdEDTA

0276058

# FIG.4

$^1$H N.M.R. OF NdEDTA INTERACTION WITH ATP (200MHz)

Solution pH 7.0; Temp. 25°C

ATP and PCr present at 60mM

10mM NdEDTA

PCr

5mM NdEDTA

0mM NdEDTA

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 071 564 (SCHERING AG)<br>* Whole document *<br>--- | 1-9 | A 61 K 49/00<br>G 01 N 24/08 |
| X | EP-A-0 169 299 (SCHERING AG)<br>* Whole document *<br>--- | 1-9 | |
| X | RADIOLOGY, vol. 147, no. 3, June 1983, pages 781-788; R.C. BRASCH: "Work in progress: methods of contrast enhancement for NMR imaging and potential applications"<br>* Page 782, table II; page 786, "Paramagnetic ions"; figure 4 *<br>--- | 1,2,5,8,9 | |
| X | MEDICAL PHYSICS, vol. 11, no. 1, January/February 1984, pages 67-72, Am. Assoc. Phys. Med., New York, US; M.A. BROWN et al.: "Transition metal-chelate complexes as relaxation modifiers in nuclear magnetic resonance"<br>* Pages 68,70 *<br>--- | 1-4,9 | TECHNICAL FIELDS SEARCHED (Int. Cl.4)<br><br>A 61 K |
| X | WO-A-8 602 005 (NEW SALUTAR, INC.)<br>* Page 13; claims 1-5 *<br>----- | 1-3,9 | |

The present search report has been drawn up for all claims

| Place of search | Date of completion of the search | Examiner |
|---|---|---|
| THE HAGUE | 08-04-1988 | ALVAREZ Y ALVAREZ C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)